(19) Europäisches Patentamt
European Patent Office
Office européen des brevets

(11) Veröffentlichungsnummer: **0 195 230**
**A2**

(12) # EUROPÄISCHE PATENTANMELDUNG

(21) Anmeldenummer: **86101648.3**

(22) Anmeldetag: **08.02.86**

(51) Int. Cl.[4]: **C 12 P 1/00**
C 12 P 19/02, C 12 P 13/14
C 12 P 7/58

(30) Priorität: **16.02.85 DE 3505397**

(43) Veröffentlichungstag der Anmeldung:
**24.09.86 Patentblatt 86/39**

(84) Benannte Vertragsstaaten:
**AT BE CH DE FR GB IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Simon, Helmut, Prof.Dr.**
**Egilbertstrasse 31**
**D-8050 Freising(DE)**

(72) Erfinder: **Deffner, Alexander**
**Römerhofweg 51 d**
**D-8046 Garching(DE)**

(54) **Verfahren zur Coenzymregenerierung.**

(57) Beschrieben wird ein Verfahren zur enzymatischen Herstellung der Coenzyme ATP, Acetyl-CoA, Acetylphosphat, NAD, NADP, beziehungsweise NADH, NADPH aus ihren physiologischen Vorprodukten, bei dem als Oxidationsmittel beziehungsweise Reduktionsmittel Alkanale wie Acidaldehyd und als Katalysator ein Zellysats eines Mikroorganismenstammes der Spezies Clostridium kluyveri eingesetzt wird.

EP 0 195 230 A2

Croydon Printing Company Ltd.

0135230

Patentanmeldung

Henkelstraße 67
4000 Düsseldorf, den 14.2.1985

Henkel KGaA
ZR-FE/Patente

Dr.Wi/Po

Patentanmeldung

D 7179 EP

"Verfahren zur Coenzymregenerierung"

Die Erfindung betrifft ein einfaches Verfahren, das es erlaubt, eine Reihe von Coenzymen wie Adenosintriphosphat (ATP), Acetyl-Coenzym A (Acetyl-CoA), Acetylphosphat, oxidiertes oder reduziertes Nikotinamid-adenindinucleotid und Nikotinamid-Adenindinucleotidphosphat während einer sie verbrauchenden biochemischen Reaktion simultan in die Ausgangsform zurückzuführen. Als Agens wird dabei lediglich ein Alkohol oder Aldehyd wie Ethanol oder Ethanal verbraucht. Katalysator ist das Lysat eines Mikroorganismus' der Spezies Clostridium kluyveri.

Enzymreaktionen gewinnen bekanntlich in der chemischen Technik immer größere Bedeutung, da sie unter milden Bedingungen ablaufen und in vielen Fällen selektiver sind als andere zur Verfügung stehende präparative Methoden. Von den bisher bekannten und in der Literatur beschriebenen Enzymen können jedoch bisher nur sehr wenige für technische Zwecke angewendet werden. So ist beispielsweise die Nutzung von Enzymen, die im Inneren von Zellen vorhanden sind und nicht das exozelluläre Stoffwechselprodukt von Einzellern darstellen, auf recht wenige Beispiele begrenzt. Unter diesen Enzymen wiederum findet die Gruppe der cosubstratabhängigen Enzyme praktisch nur in wissenschaftlichen Untersuchungen Anwendung. Unter cosubstratabhängigen Enzymen versteht man solche, die zur Katalyse einer chemischen Reaktion ein Cosubstrat (auch

...

Coenzym genannt) in stöchiometrischen Mengen mit dem Substrat verbrauchen. Der seltene Einsatz derartiger Enzyme in der Technik erklärt sich daraus, daß die Bereitstellung der Cosubstrate in großen Mengen zu aufwendig ist und daher enzymatische Verfahren, die coenzymabhängige Enzyme benötigen, im allgemeinen gegenüber anderen Synthesemethoden nicht konkurrenzfähig sind.

Um hier Abhilfe zu schaffen, hat man bereits versucht, einzelne Coenzyme zu regenerieren. So ist es beispielsweise bekant, NAD(P) in NAD(P)H zu überführen, indem man beispielsweise Lipoamiddehydrogenase (EC 1.6.4.3.) oder Extrakte von verschiedenen Mikroorganismen in Gegenwart von reduziertem Methylviologen (1,1'-Dimethyl-4,4-bipyridinium), das durch Wasserstoff und Hydrogenase oder Gleichstrom regeneriert wurde, auf NAD(P) einwirken läßt. Siehe Ch.-M. Wong et al. J. Am. Chem. Soc. 103 (1981) S. 6227 sowie J. Bader et al. J. Biotechn. 1 (1984), 95.

Wenngleich nach diesem Verfahren für eine spezielle enzymatische Reaktion die einzusetzende Menge an NA(P)DH stark begrenzt werden kann, so ist das Arbeiten in einer elektrochemischen Zelle doch relativ aufwendig. In ähnlicher Weise sind auch für andere Coenzyme wie beispielsweise Acetyl-CoA, Acetylphosphat oder ATP Reaktionen zur Regenerierung vorgeschlagen worden, die jedoch in aufwendiger Weise isolierte gereinigte Enzyme und/oder weitere, schwer zugängliche Substrate benötigen (G.M. Whitesides, Ch.-H. Wong, Aldrichimica Acta 16 (1983) 27).

Auf dem genannten Fachgebiet besteht daher ein Bedarf nach einem einfachen Regenerierverfahren für Coenzymmischungen oder einzelne Coenzyme. Die Erfinder haben sich daher die Aufgabe gestellt, ein solches Verfahren, ausgehend von Alkanolen oder Alkanalen mit 2 bis 4 C-Atomen und einer durch Lyse einer

...

Zellsuspension in einfacher Weise herstellbaren Enzymzubereitung, zur Verfügung zu stellen.

Gegenstand der Erfindung ist somit ein Verfahren zur enzymatischen Herstellung der Coenzyme Adenosintriphosphat, Acetyl-Coenzym A, Acetylphosphat, reduziertes Nikotinamid-adenindinucleotid und/oder reduziertes Nikotinamid-adenin-dinucleotid-phosphat aus ihren unter physiologischen Bedingungen auftretenden direkten Vorprodukten, dadurch gekennzeichnet, daß man die Vorprodukte einerseits sowie andererseits als Reduktionsmittel primäre Alkanole und/oder Alkanale mit je 2 bis 4 C-Atomen oder als Oxidationsmittel Alkanale mit 2 - 4 C-Atomen in Gegenwart eines Zellysats eines Mikroorganismenstammes der Spezies Clostridium kluyveri und gewünschtenfalls in Gegenwart weiterer Enzyme, reagieren läßt.

Mikroorganismen der Spezies Clostridium kluyveri sind seit langem bekannt. Es handelt sich dabei um anaerobe Bakterien, die in der Lage sind, Ethanol und Acetat zu Butyrat, Caproat und molekularem Wasserstoff umzuwandeln. In der wissenschaftlichen Literatur sind auch zahlreiche endozelluläre Enzyme verschiedener Stämme dieser Spezies beschrieben. So beschreiben beispielsweise R.M. Burton et. al. die Oxidation von Acetaldehyd zu Acetyl-Coenzym A (J. Biol. Chem. 202 (1953), S. 873). Von R. Lurz (Arch. Microbiol. 120 (1979), S. 255) wird die Isolierung eines speziellen Alkohol-Acetaldehyddehydrogenase-Komplexes aus Clostridium kluyveri beschrieben. Schließlich wird von J. Bader et. al. (Arch. Microbiol. 127 (1980), S. 279) die Hydrierung alpha-beta-ungesättigter Carbonsäureverbindung mit Hilfe eines Lysats von Clostridium kluyveri beschrieben. Wenn somit für den Fachmann bekannt war, daß der genannte Mikroorganismus über Enzyme verfügt, mit deren Hilfe auch Coenzyme regeneriert werden können, und wenn auch bereits bei speziellen stereoselektiven Hydrierungen mit dem Lysat von Clostridium kluyveri gearbeitet wurde, so ist es doch überraschend, daß

...

derartige Lysate Biokatalysatoren darstellen, die eine für einen technischen Prozess ausreichende Stabilität der Enzymaktivität aufweisen und die über derartig komplexe Enzymaktivitäten verfügen, daß mehrere Coenzyme, wie beispielsweise ATP, Acetyl-CoA, Acetylphosphat und NAD(P)H nebeneinander regeneriert werden können. Wenngleich die überraschende Stabilität der Enzymaktivität des Lysats wissenschaftlich nicht begründet werden kann, so ist doch anzunehmen, daß sie in der Besonderheit begründet wird, daß der Mikroorganismus Clostridium kluyveri nicht über Proteasen verfügt und auch nicht oder höchstens in untergeordneter Menge über Enzyme, die Adenosinnukleotide und/oder Pyridinnukleotide angreifen. So wären beispielsweise Lysate von Hefezellen der Spezies Saccharomyces cerevisiae oder Candida utilis für das erfindungsgemäße Verfahren trotz einer ähnlichen Enzymausstattung ungeeignet, da hier proteolytische Enzyme vorhanden sind.

Mit Hilfe des erfindungsgemäßen Verfahrens ist es summarisch möglich, die Coenzyme ATP, Acetyl-CoA, Acetylphosphat und NAD(P)H unter Oxidation eines Alkanols oder Alkanals mit 2 bis 4 C-Atomen, insbesondere Ethanol oder Ethanal, zu regenerieren. Sollen NAD bzw. NADP regeneriert werden, so ist der Einsatz von Alkanalen, insbesondere Ethanal erforderlich. Bei der Regenerierung mehrerer Coenzyme sowie bei der Regenerierung von NAD(P)H alleine wird bevorzugt Ethanol eingesetzt. Vereinfacht lassen sich die Regenerierungsreaktionen durch das folgende Schema verdeutlichen: (Die durchgezogenen Pfeile zeigen die Reaktionen zur Regenerierung von NAD(P)H, Acetyl-CoA, Acetyl-phosphat oder ATP. Die gestrichelten Pfeile ergeben den Reaktionsverlauf zur Regenerierung von NAD(P)).

. . .

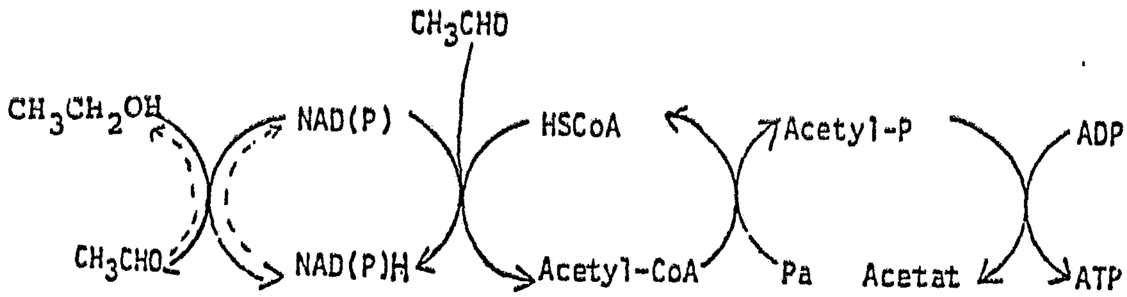


Zur Herstellung des dabei als Katalysator verwendeten Zellysats geht man von einer Zellkultur eines Stammes von Clostridium kluyveri aus. Geeignete Stämme sind insbesondere der Stamm Clostridium kluyveri DSM 555 (ATCC 8527) bzw. ATCC 12489. Weiter geeignet sind auch die Stämme DSM 556, 557, 560, 562, 563 und 564. Zur Auswahl besonders geeigneter Stämme wird der Fachmann in jedem Fall die Fähigkeit der Lysate zur Coenzymregenerierung, wie in den nachfolgenden Beispielen geschildert, überprüfen. Dabei sollten die mit dem Stamm DSM 555 erreichten Werte, bezogen auf Enzymeinheiten, zu mindestens zu 20 %, vorzugsweise zu mindestens 60 %, erreicht werden.

Zur Gewinnung der Zellysate wird zunächst eine Kultur eines Stammes Clostridium kluyveri in bekannter Weise hergestellt. Die Anzucht erfolgt unter Sauerstoffausschluß. Es können dabei die in der Literatur bekannten Medien verwendet werden. So eignet sich beispielweise ein von Stadtman et. al. beschriebenes Medium (In: Methods in enzymology, Vol. 1. S.P. Colowick, N.O. Kaplan, Eds., pp. 518 - 523, New York : Academic Press (1955). Weiterhin geeignet sind auch Medien, die von J. Bader et. al. in Hoppe-Seyler's Z. Physiol. Chem. Bad. 359, S. 20 beschrieben worden sind. Danach verwendet man 16 ml Ethanol, 7,5 g Natriumacetat, 2,5 g Eisessig, 150 mg Diammoniumhydrogenphosphat, 100 mg Dikaliumhydrogenphosphat,

...

33 mg Magnesiumchlorid-6-$H_2O$, 0,6 mg Magnesiumsulfat-7-$H_2O$, 40 mg Calciumchlorid-2-$H_2O$, 0,4 mg Mangansulfat-2-$H_2O$, 0,4 mg Eisensulfat-7-$H_2O$, 50 mg Ammoniumchlorid, 10 mg Ammoniumolydat-4-$H_2O$, 0,04 mg Biotin, 0,8 mg Paraaminobenzoesäure, 1 mg Resazurin, 6 ml 50 %ige Lösung von Kaliumcarbonat und 1 Liter demineralisiertes Wasser. Das Zuchtmedium wird in geeigneter Weise sauerstofffrei gemacht und bei 37°C beimpft. Während des Zellwachstums kann gerührt werden. Es ist von Vorteil, ein sterilisiertes, sauerstofffreies Gas, wie etwa Stickstoff, durch die Ansätze zu leiten. Zum Ernten der Zellen wird vorzugsweise zentrifugiert. Falls Waschen gewünscht wird, wird die Zellmasse bei 0°C unter Stickstoff in einer 0,05-molaren Phosphatpufferlösung von pH 7, die 0,14 mol Natriumchlorid und 1 mg Resazurin pro Liter enthält, aufgeschlämmt. Dabei kann pro Liter 10 mg Natriumdithionit zugegeben werden. Bezüglich weiterer Einzelheiten sei auf die oben zitierte Literaturstelle von J. Bader et. al. verwiesen.

Zur Herstellung eines Zellysats werden die frisch geernteten Zellen oder auch unter Tiefkühlbedingungen (Temperaturen $<-10°C$) eingefrorene und wieder aufgetaute Zellsuspensionen unter Ausschluß von Sauerstoff einer Behandlung unterzogen, bei der die Zellmembranen zerstört werden. Die Zerstörung der Zellmembranen wird vorzugsweise enzymatisch durchgeführt. Denkbar ist jedoch auch eine mechanische Zerstörung, beispielsweise durch Ultraschall. In speziellen Fällen kann auch eine chemische oder physikalisch-chemische Zerstörung vorweggenommen werden, etwa durch Zugabe oberflächenaktiver Substanzen. Dies ist jedoch weniger bevorzugt. Da Zellysate aufgrund des hochmolekularen Charakters der in ihnen vorhandenen DNS viskose Flüssigkeiten darstellen, die für viele Anwendungen wenig geeignet sind, ist es empfehlenswert, zur Viskositätsminderung die DNS zu spalten. Dies wird vorzugsweise durch DNS-spaltende Enzyme (DNasen) durchgeführt.

...

Zur Herstellung besonders geeigneter Zellsuspensionen werden die Zellen vorzugsweise in einer Pufferlösung, insbesondere einer Phosphatpufferlösung, mit einer Konzentration von 0,1 - 0,5 mol pro Liter aufgeschlämmt. der pH dieser Lösung beträgt 6 bis 8, vorzugsweise um 7. So kann insbesondere mit Pufferlösungen, die einen pH-Wert in der Bandbreite von 6,75 bis 7,5 haben, gearbeitet werden. Die Enzyme, die die Zellwand spalten, werden in Mengen von 5 bis 30 mg, vorzugsweise 15 bis 20 mg pro Gramm feuchtes Zellmaterial, eingesetzt. Bevorzugt sind hier handelsübliche Lysozyme, z.B. das Produkt Lysozym (Hydrochlorid) der Firma Boehringer, Mannheim. Gemeinsam mit oder nach der Lysozymbehandlung werden 0,5 bis 3 mg, vorzugsweise 1,5 bis 2 mg, einer handelsüblichen DNase pro Gramm feuchtes Zellmaterial zugegeben. Die Lysate werden bei Temperaturen zwischen 25 und 40$^{o}$C, insbesondere bei Temperaturen um 37$^{o}$C, also zwischen 35 und 37$^{o}$C, hergestellt. Die Reaktionszeiten für den enzymatischen Prozess der Zellspaltung und des DNas-Abbaus betragen 20 bis 90 Minuten, vorzugsweise 60 - 80 Minuten. Zur Steigerung der Aktivität kann gewünschtenfalls bei 0$^{o}$C eine Ultraschallbehandlung angeschlossen werden. Für kleine Probemengen, also zum Beispiel ein Lysat auf Basis von einem Gramm feuchter Zellen, beträgt dabei die Beschallungsdauer 2 Minuten in einem 30-Watt-Gerät. In vielen Fällen ist es wünschenswert, den Lysaten Antibiotika zuzusetzen. So kann pro Gramm Zellmaterial ein Milligramm Tetracyclin oder dessen HCl-Salz zugegeben werden.

Die so hergestellten Zellysate zeigen eine hohe Enzymaktivität. Erfindungsgemäß werden sie zur Regenerierung von Coenzymen eingesetzt. Dabei kann beispielsweise ATP aus AMP (Adenosinmonophosphat) in Gegenwart katalytischer Mengen von ADP oder insgesondere aus ADP (Adenosindiphosphat) hergestellt werden. Eine weitere Reaktion ist die Regenerierung von Acetyl-Coenzym A aus beispielsweise Acetaldehyd und HS-CoA oder Coenzym A. Weiterhin können NADPH und/oder NADH aus NADP bzw. NAD

...

hergestellt werden. Hergestellt werden kann aber auch NAD(P) aus NAD(P)H. Aufgrund der Fähigkeiten des verwendeten Biokatalysators ist es möglich, die Regenerierungen von NAD(P)H und ATP oder Acetyl-CoA bzw. Acetylphosphat simultan durchzuführen. Die genannten Cosubstrate können auch einzeln regeneriert werden. Wird z.B. nur ATP-Regenerierung gewünscht, so sind auch nur katalytische Mengen von NAD erforderlich, da das gebildete NADH unter Bildung von Ethanol wieder zu NAD reoxidiert werden kann.

Bei den genannten Regenerierungsreaktionen wird ein Hilfssubstrat als Reduktionsmittel verbraucht. Das erfindungsgemäße Verfahren erlaubt es, als Hilfssubstrate ein Alkanol oder ein Alkanal mit 2 bis 4 C-Atomen einzusetzen und zwar ein Alkanol oder Alkanal als Reduktionsmittel für NAD(P) bzw. ein Alkanal als Oxidationsmittel für NAD(P)H. Wenngleich das Verfahren mit n-Butanol, n-Butanal, n-Propanol, n-Propanal prinzipiell durchgeführt werden kann, so ist es doch in jedem Falle bevorzugt, Ethanol und/oder Acetaldehyd einzusetzen. Die Reduktionsmittel Alkanol und/oder Alkanal bzw. das Oxidationsmittel Alkanal werden dabei in leichtem stöchiometrischem Überschuß, bezogen auf die unter Coenzymregenerierung umzusetzende Substrate angewandt. Dabei werden die vorhandenen Coenzymmengen mehrfach, beispielsweise einige hundert Mal, regeneriert. Zweckmäßigerweise wird ein Überschuß an Reduktions- bzw. Oxidationsmittel von 10 - 50 mol-% oder gewünschtenfalls ein sehr hoher Überschuß von mehreren hundert mol-% eingesetzt.

Bei länger laufenden Anwendungen des erfindungsgemäßen Verfahrens hat der Fachmann dafür Sorge zu tragen, daß Säuren, die als Oxidationsprodukte des Reduktionsmittels auftreten, den Reaktionsverlauf nicht stören. Es wird daher in Pufferlösungen geeigneter Stärke gearbeitet. Bevorzugt sind Pufferlösungen mit einem pH zwischen 6 und 8, insbesondere

...

zwischen 6,75 und 7,5. Geeignete Pufferlösungen sind Phosphatpuffer oder andere physiologisch verträgliche Puffer. So können beispielsweise Puffer auf Basis des Natriumsalzes der Morpholinopropansulfonsäure (MOPS) eingesetzt werden, insbesondere falls Acetyl-CoA regeneriert werden soll. Prinzipiell verwendbar, aber für manche Reaktionen wenig geeignet, sind Puffer auf Basis von 2-Amino-2-hydroxymethyl-1,3-propandiol (TRIS$^R$) und anorganischen Säuren wie HCl.

Das erfindungsgemäße Verfahren benötigt das Lysat eines Mikroorganismus' der Spezies Clostridium kluyveri nur in katalytischen Mengen. So werden beispielsweise pro mmol unter Coenzymregenerierung zu bildendes Produkt in 4 - 7 Stunden nur 2 - 50 mg, vorzugsweise nur 20 - 40 mg Lysatprotein (bestimmt nach der Methode von Bradford) eingesetzt (Literatur: S.M. Read, D.H. Northcofe, Anal.Biochem. 116 (1981), S. 53). Für längere Reaktionszeiten können entsprechend höhere Mengen verwendet werden.

Die Aktivität des Lysats von Clostridium kluyveri ist durch die Acetatkinase-Aktivität begrenzt. Daher wird nach einer weiteren Ausführungsform der Erfindung zusätzlich Acetatkinase zugegeben. So können beispielsweise pro mmol unter ATP-Verbrauch umgesetztes Substrat 5 - 20, vorzugsweise 10 - 15 U Acetatkinase zugegeben werden. Die Einheit U ist so definiert, daß 1 U Enzym unter physiologischen Bedingunen 1 µmol Substrat pro Minute umsetzt. Geeignete Acetatkinasen (EC2.7.2.1.) können beispielsweise aus E.coli erhalten werden und sind als solche im Handel (Fa. Boehringer, Mannheim). Darüber hinaus können Acetatkinasen aus beliebigen anderen Mikroorganismen eingesetzt werden, wenn sie über die entsprechende Aktivität verfügen.

Das erfindungsgemäße Verfahren kann zur präparativen Herstellung der Coenzyme eingesetzt werden. Von besonderem Wert ist jedoch, daß es erfindungsgemäß möglich ist, die an sich schwer

. . .

zugänglichen Coenzyme bei Reaktionen, in denen sie verbraucht werden, nur in katalytischen Mengen einzusetzen und während der Verbrauchsreaktion mit Hilfe des Lysats von Clostridium kluyveri und des Reduktionsmittels, also beispielsweise Ethanol oder Ethanal, kontinuierlich zu regenerieren. So kann während einer solchen Reaktion das Coenzym ATP und/oder die Coenzyme NAD(P)H 300mal oder auch weitaus häufiger regeneriert werden. Dies gilt auch für Acetyl-CoA und/oder Acetylphosphat für sich alleine oder Mischungen mit den vorgenannten Coenzymen. Dabei hat sich gezeigt, daß das erfindungsgemäß eingesetzte Lysat einen Enzymkatalysator darstellt, also keine Mischung, die nur aufgrund ihrer stationären Konzentration an regenerierten Coenzymen wirksam ist. Falls das NAD(P)H nicht für die gewünschte ATP verbrauchende Reaktion ebenfalls verbraucht wird, wird NAD(P) unter Ethanolbildung (aus Acetaldehyd) regeneriert.

In den folgenden Beispielen werden coenzymabhängige Reaktionen beschrieben. Aus der Reaktionskinetik ergibt sich, daß die in katalytischen Konzentrationen eingesetzten Coenzyme durch das zugegebene Lysat von Clostridium kluyveri DSM 555 und Ethanol bzw. Ethanol kontinuierlich regeneriert werden. Als Beispiele für die intermediäre ATP-Regenerierung wurde die Darstellung von Glucose-6-phosphat und Glycerin-3-.phosphat durchgeführt.
Für die NADH- bzw. NADPH-Regenerierung wurde die reduktive Aminierung von 2-Oxoglutarat zu Glutamat und für die NAD(P)-Regenerierung wurde die Dehydrierung von Glucose-6-phosphat zu 6-Phosphogluconat bzw. von Glucose zu Gluconat bewerkstelligt. Es werden die in der biochemischen Fachliteratur gebräuchlichen Abkürzungen verwendet.

...

# B e i s p i e l e

## Beispiel 1:

Darstellung von Glucose-6-Phosphat (G-6-P)

Allgemeiner Ansatz:
Bedingungen, falls nicht anders angegeben: Temp. 36°C, Stickstoffatmosphäre, 0,3 M Kaliumphosphat, pH 7; Gesamtvolumen 1 ml; Parallel-Ansätze in Rollfläschchen mit Septenverschluß im Warburg-Schüttler. Die Proteinbestimmungen erfolgten nach Bradford.

Inkubiert wurden in Puffer: HSCoA 0,3 mM, NAD(P) 1,5 mM, [1) ] ADP 3,0 mM, $MgCl_2$ 2,5 mM, Tetracyclin 15 µl (1,5 mg/ml), Lysat C.klyveri DSM 555 3,2 mg Protein (nach Bradford-Test), Glucose 150 mM, 4 U Acetatkinase (EC 2.7.2.1) (E.coli, Fa. Böhringer), ca. 4 U Hexokinase (EC 2.7.1.1) (Hefe, Fa. Böhringer). Stündlich wurden 30 µl einer Lösung von 1.78 M $CH_3CHO$ in 1 M $K_2HPO_4$ zugegeben. Die Ansätze enthalten ferner 50 - 60 mM $(NH_4)_2SO_4$, da die 20 zugesetzten Enzyme Ak, Hk (Acetatkinase und Hexosekinase) als Kristallsuspension in 3.2. M $NH_4SO_4$ geliefert werden.

Ein Ansatz mit stündlicher Zugabe von 45 - 50 mM Acetaldehyd ist in Tabelle 1 gezeigt. Die katalytischen Mengen von 0,3 mM HSCoA werden bei der ATP-Regenerierung ca. 330-fach regeneriert und das NAD ca. 67-fach.

1) Statt Mengen werden Konzentrationen angegeben, die sich auf eine einheitliche Ansatzgröße beziehen.

. . .

## Tabelle 1

**Bildung von G-6-P in Gegenwart von 1,5 mM NAD bzw. NADP**

Konzentration an G-6-P nach bestimmten Zeiten bei Mitverwendung von NAD bzw. NADP

| Zeit | NAD | NADP |
|---|---|---|
| 0 h | < 3 mM | < 3 mM |
| 2 h | 45 mM | 41 mM |
| 3 h | - | 50 mM |
| 4 h | 70 mM | 73 mM |
| 5 h | 75 mM | 77 mM |
| 6 h | - | 95 mM |
| 7 h | 103 mM | 100 mM |

Beispiel 2:

Ansatz mit kontinuierlicher Substratzugabe ($CH_3CHO$):

Allgemeine Bedingungen: $V_{Anfang}$ = 3 ml, $V_{Ende}$ = 4 ml, Temp. 37°C, Stickstoffatmosphäre, Magnetrührer, KPP 300 mM.

Inkubiert wurden: NAD 1.66 mM, ADP 3,33 mM, HSCoA 0.5 mM, $NH_4SO_4$ 85 mM, $MgSO_4$ 10 mM, Tetracyclin 50 µl (1.5 mg/ml), Glucose 350 mM, $CH_3CHO$: 4 M $CH_3CHO$ in $K_2HPO_4$ (2 M), Flow 50 µl/h, Hk (Hefe) ca. 40 U (Hefe, Fa. Boehringer), Ak (E.coli) ca. 20 U (E.coli), Fa. Boehringer), Lysat Clostridium kluyveri DSM 555 : 600 ul - 9.72 mg Protein im Aufschluß.

Nach 18.5 h konnten 195 mM G-6-P im Ansatz nachgewiesen werden. (Nachweismethode: Enzymatisch nach G. Lang und G.

...

Michal in H.U. Bergmeyer (Hrsg.) Meth. der enzym. Analyse, 3. Auflage, Band II, 1283, Verlag Chemie, Weinheim, 1974).

Beispiel 3:

Bildung von Acetyl-CoA
Bedingungen: Temp. 36°C, Stickstoffatmosphäre, Gesamtvolumen 1 ml, 0.1 M Mops-Puffer, pH 7.5 (Fa. Sigma)

Inkubiert wurden: NAD 1.25 mM, HSCoA 20 mM, Lysat C.kluyveri DSM 555 - 3.5 mg Protein/ml, (Lysozym-Aufschluß in Mops-Puffer 0.1 molar, pH 7.5), $CH_3CHO$: 50 mM (30 µl einer $CH_3CHO$-Lösung - 1 : 10 in Mops, pH 7.5 verdünnt). Nach 14.5 h konnten 19.75 mM (98 % Ausbeute) CoA-Ester nachgewiesen werden (Hydroxamsäure-Test).

Beispiel 4:
NADH und NADPH-Regenerierung ohne gleichzeitige ATP-Bildung.
Dabei wird von folgenden Reaktionen Gebrauch gemacht:

$$C_2H_5OH + 2\ NAD(P) \xrightarrow{C.kluyveri} CH_3COOH + 2NAD(P)H$$

$$2NAD(P)H + 2\text{-Oxoglutarat} + 2NH_3 \xrightarrow{Glut\text{-}DH} 2\ Glutamat + NAD(P)$$

$$\text{Summe: } C_2H_5OH + 2\text{-Oxoglutarat} + 2NH_3 \longrightarrow 2\ Glutamat + CH_3COOH$$

Der Ansatz enthielt in mM Konzentrationen: 300 Kaliumphosphat pH 8, 1.5. NAD oder NADP, 0.3 HSCoA, 3.0 ADP (zur Aktivierung der Glutamatdehydrogenase), 100 2-Oxoglutarat, 200 Ammoniumsulfat, 1.2 Mangansulfat, 688 Ethanol, 0.022 mg Tetracyclin/ml, 15 U Glutamat-Dehydrogenase und Lysat von C.kluyveri DSM 555 entsprechend 3.5 mg Protein.

. . .

Unter den hier angegebenen Bedingungen kann der Zusatz von Coenzym A (HSCoA) unterbleiben. Die Reaktionsgeschwindigkeit ist mit und ohne Coenzym A gleich.

Die Bildung von Glutamat in Gegenwart katalytischer Mengen NAD ist in Tabelle 2 gezeigt.

Dieses Experiment zeigt folgendes: NADH und NADPH können mit dem System regeneriert werden. Dazu ist es nicht notwendig, das entstehende Acetyl-P durch irgendwelche zusätzliche Maßnahmen zu spalten. Die katalytischen Mengen von 1.5 mM NAD werden bei der NADH-Regenerierung 67-fach cyclisiert, ohne daß die Aktivität des Lysats nachließ.

T a b e l l e 2

Bildung von Glutamat unter Regenerierung von:

| Zeit | NADH | NADPH |
|---|---|---|
| 0 h | < 2 mM | < 2 mM |
| 1 h | 31 mM | 13 mM |
| 2 h | 52 mM | 25 mM |
| 3 h | 70 mM | 34 mM |
| 4 h | 80 mM | 40 mM |
| 5 h | - 100 mM | 46 mM |

Beispiel 5:

Gleichzeitige Regenerierung von NAD(P)H und ATP

Die Tabelle 3 zeigt, daß ATP und NADH gleichzeitig regeneriert werden können. Dieser Ansatz enthielt zusätzlich zu dem in Beispiel 2 genannten 100 mM Glucose, 15 U Hexokinase, 2 mg Protein eines Rohlysats von Clostridium sporogenes ATCC 3584 und im Abstand von einer Stunde wurde 50 mM Acetaldehyd zugegeben. NADPH-Regenerierung verläuft analog.

. . .

Tabelle 3

Bildung von Glutamat und G-6-P unter gleichzeitiger Regenerierung von NADH und ATP:

| Zeit | Glutamat | G-6-P |
|---|---|---|
| 0 h | < 2 mM | < 2 mM |
| 1 h | 49 mM | 17,5 mM |
| 2 h | 85 mM | 26 mM |
| 3 h | 85 mM | - |
| 4 h | - | 38 mM |

Beispiel 6:

Die Regenerierung von NADP

Diese Regenerierung ist ebenfalls durch das System Acetaldehyd/Rohextrakt C.kluyveri möglich. Dies wurde durch die Reaktionsfolge:

$$\text{Gluc-6-P+NADP} \xrightarrow[\text{Dehydrogenase}]{\text{Gluc-6-P}} \text{6-Phosphogluconat + NADPH}$$

$$\text{NADPH+}CH_3CHO\text{+}H^+ \xrightarrow{\text{C.kluyveri DSM 555}} CH_3CH_2OH \text{ + NADP}$$

$$\text{Summe: Gluc-6-P+}CH_3CHO \longrightarrow \text{6-Phosphogluconat + } CH_3CH_2OH$$

bestätigt.

Tabelle 4 zeigt den zeitlichen Verlauf der Dehydrierung von Glucose-6-phosphat. Der Ansatz enthielt in mM Konzentrationen: 200 Kaliumphosphatpuffer pH 7.0, 1.5 NADP, 100 Glucose-6-phosphat, 60 mM Acetaldehyd, 2 mg Protein/ml und 5 U Glucose-6-phosphat-Dehydrogenase/ml.

...

## Tabelle 4

Abnahme von G-6-P unter Regenerierung von NADP:

| Zeit | G-6-P | Inkubation von $CH_3CHO$ |
|---|---|---|
| 0 h | 100 mM | + 60 mM |
| 1/2 h | 40 mM | - |
| 1 h | 39 mM | + 60 mM |
| 3 h | 4 mM | - |
| 4 h | ≤ 1 mM | - |

Beispiel 7:

Die Regenerierung von NAD:
Die Regenerierung wurde durch folgende Reaktionsfolge nachgewiesen:

$$NAD + Glucose \xrightarrow[\text{Dehydrogenase}]{\text{Glucose-}} NADH + Gluconsäure$$

$$NADH + CH_3CHO \xrightarrow{\text{C.kluyveri DSM 555}} NAD + CH_3CH_2OH$$

Tabelle 5 zeigt den zeitlichen Verlauf der Dehydrierung von Glucose. Unter Bedingungen des allgemeinen Ansatzes wurden folgende Konzentrationen in mM inkubiert:

200 Kaliumphosphatpuffer pH 7.0, 1 NAD, 100 Glucose, 20 U Glucose-Dehydrogenase (EC 1.1.1.47) (Fa. Merck), 2.7 mg Protein (Lysat C. kluyveri), $CH_3CHO$ 53 mM zur Zeit t = 0 h und t = 1/2 h.

...

## Tabelle 5

Abnahme von Glucose unter gleichzeitiger Regenerierung von NAD:

| Zeit | Glucose |
|---|---|
| 0 h | 100 mM |
| 1/2 h | 49 mM |
| 1 h | ~ 1 mM |

## Patentansprüche

1. Verfahren zur enzymatischen Herstellung der Coenzyme

   - Adenosintriphosphat
   - Acetyl-Coenzym A
   - Acetylphosphat
   - reduziertes Nikotinamid-adenin-dinucleotid
   - reduziertes Nikotinamid-adenin-dinucleotidphosphat
   - oxidiertes Nikotinamid-adenin-dinucleotid und/oder
   - oxidiertes Nikotinamid-adenin-dinucleotidphosphat

   aus ihren unter physiologischen Bedingungen auftretenden direkten Vorprodukten, dadurch gekennzeichnet, daß man die Vorprodukte einerseits sowie andererseits als Reduktionsmittel primäre Alkanole und/oder Alkanale mit je 2 bis 4 C-Atomen oder als Oxidationsmittel Alkanale mit 2 - 4 C-Atomen in Gegenwart eines Zellysats eines Mikroorganismenstammes der Spezies Clostridium kluyveri und gewünschtenfalls in Gegenwart weiterer Enzyme, reagieren läßt.

2. Verfahren nach Anspruch 1, dadurch gekennzeichnet, daß man als Zellysat eine Aufschlämmung von Zellen mit enzymatisch, mechanisch oder chemisch gespaltenen Zellwänden und gewünschtenfalls durch DNase depolymerisierter DNS einsetzt.

3. Verfahren nach den Ansprüchen 1 und 2, dadurch gekennzeichnet, daß man ein Zellysat einsetzt, das durch Aufschlämmung von Zellen eines Mikroorganismus' der Spezies Clostridium kluyveri, die vorzugsweise frisch geerntet sind, in einer Phosphatpufferlösung der Konzentration 0,1 bis 0,5 mol pro Liter bei einem pH von 6 bis 8, vorzugsweise um 7, in Gegenwart von 5 bis 30 mg, vorzugsweise von 15 bis 20 mg eines handelsüblichen Lysozyms und 0,5 bis 3 mg,

...

vorzugsweise 1,5 bis 2 mg einer handelsüblichen DNase, jeweils bezogen auf 1 g feuchtes Zellmaterial, bei Temperaturen zwischen 25 und 40°C, vorzugsweise 35 - 37°C, und Reaktionszeiten zwischen 20 und 90 Minuten, vorzugsweise 60 - 80 Minuten, aufgeschlossen worden ist und gewünschtenfalls bei 0°C einer Ultraschallbehandlung unterzogen worden ist.

4. Verfahren nach den Ansprüchen 1 bis 3, dadurch gekennzeichnet, daß man Adenosintriphoshat (ATP) in Mischung mit reduziertem Nokotinamid-adenin-dinucleotid (NADH) und/oder reduziertem Nikotinamid-adenin-dinucleotid-Phosphat (NADPH) und gewünschtenfalls Acetylphosphat und/oder Acetyl-Coenzym A (Acetyl-CoA) herstellt.

5. Verfahren nach den Ansprüchen 1 - 4, dadurch gekennzeichnet, daß NAD(P)H unter Ethanolbildung zu NAD(P) umgesetzt wird.

6. Verfahren nach den Ansprüchen 1 bis 5, dadurch gekennzeichnet, daß man als Alkanol Ethanol und als Alkanal Acetaldehyd im stöchiometrischen Überschuß, bezogen auf das zu bildende Coenzym oder das umzusetzende Substrat, einsetzt.

7. Verfahren nach den Ansprüchen 1 bis 6, dadurch gekennzeichnet, daß man als Mikroorganismus der Spezies Clostridium kluyveri einen der Stämme Clostridium kluyveri ATCC 8527, ATCC 12489, DSM 556, DSM 557, DSM 560, DSM 562, DSM 563, DSM 564, insbesondere DSM 555, einsetzt.

8. Verfahren nach den Ansprüchen 1 bis 7, dadurch gekennzeichnet, daß man das Lysat von Clostridium kluyveri in solchen Mengen einsetzt, daß pro mmol zu regenerierendem

...

Coenzym 2 - 50, vorzugsweise 20 - 40 mg Lysatprotein (bestimmt nach der Bradford-Methode) vorhanden sind.

9. Verfahren nach den Ansprüchen 1 bis 8, dadurch gekennzeichnet, daß in Pufferlösungen mit einem pH von 6 bis 8, vorzugsweise 6,75 bis 7,5m gearbeitet wird.

10. Verfahren nach den Ansprüchen 1 bis 9, dadurch gekennzeichnet, daß als weitere Enzyme handelsübliche Acetokinasen in Mengen von 5 bis 20 U pro mmol zu regenerierendem ATP oder zu phosphorylieredenm Substrat eingesetzt wird.

11. Verwendung des Verfahrens nach den Ansprüchen 1 bis 10 zur kontinuierlichen Bereitstellung der Coenzyme ATP, Acetyl-CoA, Acetylphosphat, NADPH, NADH, NAD, NADP oder deren Mischungen, wobei Mischungen von NAD(P) mit NAD(P)H ausgenommen sind, in einem diese Coenzyme verbrauchenden Reaktionsgemisch unter physiologischen Bedingungen.

. . .